# EUROPEAN PATENT APPLICATION

(11) **EP 4 589 596 A1**
(43) Date of publication of application: **23.07.2025**
(21) Application number: 24838630.2
(22) Date of filing: 01.07.2024
(51) Int. Cl.: G16B 30/10, G16B 30/20, G16B 40/20, G06F 18/214, G06F 18/241, C12Q 1/6869, C12M 1/34

(54) **FLOW CYTOMETRY SEQUENCING ANALYSIS METHOD AND APPARATUS, STORAGE MEDIUM AND COMPUTER DEVICE**

(30) Priority: 10.07.2023 CN 202310834707
(71) Applicant: BGI Tech Solutions Co., Ltd, Shenzhen, Guangdong 518000 (CN)
(72) Inventor: YANG, Jiaobo, Shenzhen, Guangdong 518000 (CN); ZHANG, Youjin, Shenzhen, Guangdong 518000 (CN); HE, Zengquan, Shenzhen, Guangdong 518000 (CN); WANG, Jinan, Shenzhen, Guangdong 518000 (CN); JIN, Xiangqian, Shenzhen, Guangdong 518000 (CN)
(74) Representative: Potter Clarkson
(86) International application number: PCT/CN2024/102851
(87) International publication number: WO 2025/011375

(57) **Abstract**

Provided are a flow sequencing and analysis method and an apparatus, a storage medium, and a computer device, which relate to the technical field of gene sequencing. After a to-be-analyzed sample and a corresponding analysis demand thereof and idle conditions of all current sequencers are acquired, a first sequencer using a second generation sequencing technology and a second sequencer using a third generation sequencing technology have different advantages, and therefore, a target sequencer can be determined in conjunction with the sample property of the to-be-analyzed sample and the analysis demand of the to-be-analyzed sample and the idle conditions of all the current sequencers. The target sequencer can be the first sequencer or the second sequencer, or can use the first sequencer and the second sequencer for mixed sequencing. **In** this way, not only diversified use scenarios can be provided for users, but also the sequencing efficiency and precision can be improved to a certain extent. The to-be-analyzed sample can be sequenced by means of the target sequencer to obtain a base sequence corresponding to the to-be-analyzed sample, where the base sequence is a binary sequence.

## Description

The present application claims priority to Chinese Patent Application No. 202310834707.1, filed to China National Intellectual Property Administration on July 10, 2023, entitled "FLOW SEQUENCING AND ANALYSIS METHOD AND APPARATUS, STORAGE MEDIUM, AND COMPUTER DEVICE", the entire content of which is incorporated herein by reference.

### TECHNICAL FIELD

The present disclosure relates to the technical field of gene sequencing, in particular, to a flow sequencing and analysis method, a flow sequencing and analysis apparatus, a storage medium, and a computer device.

### BACKGROUND

A DNA sequencing technology has played a vital role in promoting the development of molecular biology since its invention. In 1977, Walter Gilbert and Frederick Sanger invented a first sequencer and used the first sequencer to determine a first genome sequence, phage X174, with a total length of 5375 bases. Since then, human beings have gained the ability to explore the genetic nature of life, the research of life science has entered the era of genomics, and in the past 40 years, a sequencing technology has been considerably developed from a first generation sequencing technology to a third generation sequencing technology. The first generation sequencing technology is a sequencing method invented by Sanger, this technology has been widely used until now, but a sequence with a length of 700-1000 bases can be only obtained by using the first generation sequencing technology at a time, which cannot meet urgent needs of modern scientific development for biological gene sequence acquisition. Next Generation Sequencing (NGS) or second generation sequencing is High-Throughput Sequencing (HTS), which is a revolutionary change to traditional Sanger sequencing and solves the limitation that first generation sequencing technology can only determine one sequence at a time. The HTS can obtain hundreds of thousands to millions of nucleic acid sequences at the same time by one-time operation. For the second generation sequencing technology, the sequencing throughput is greatly increased, but the obtained single sequence is very short, and accurate gene sequence information depends on higher sequencing coverage and an accurate sequence splicing technology, therefore, final results obtained by the second generation sequencing technology have certain error information. Therefore, researchers have invented a third generation sequencing technology also known as single-molecule sequencing technology, by which de novo sequencing is performed on a single long sequence on the basis of ensuring the sequencing throughput, and thus, information of a nucleic acid sequence with a length of tens of thousands of bases can be directly obtained.

After sequencing is completed by means of a sequencer using the above-mentioned sequencing technology, matched gene sequencing and analysis software is required to analyze and interpret data. At present, due to a huge gene data volume, a relatively single use scenario and high dependence on a server in an analysis process, when a large number of samples are inputted in parallel, it is easy to cause I/O congestion, and a thread or process may be suspended, which greatly affects the analysis efficiency and the analysis precision.

### SUMMARY

Objects of the present disclosure are to solve at least one of the above-mentioned technical problems, and particularly solve the technical defect that due to a huge gene data volume, a relatively single use scenario and high dependence on a server in an analysis process, when a large number of samples are inputted in parallel, it is easy to cause I/O congestion, and a thread or process may be suspended, which greatly affects the analysis efficiency and the analysis precision.

The present disclosure provides a flow sequencing and analysis method. The method includes: acquiring a to-be-analyzed sample, an analysis demand of the to-be-analyzed sample, and idle conditions of all current sequencers; determining a target sequencer according to a sample property of the to-be-analyzed sample, the analysis demand of the to-be-analyzed sample and the idle conditions of all the current sequencers, and sequencing the to-be-analyzed sample by means of the target sequencer to obtain a base sequence corresponding to the to-be-analyzed sample, where the base sequence is a binary sequence; and inputting the base sequence to an internal storage, and invoking an analysis mode corresponding to a species to which the to-be-analyzed sample belongs and a sequencing technology used by the target sequencer to analyze the base sequence and generate a sequencing report.

Optionally, all the current sequencers include a first sequencer using a second generation sequencing technology and/or a second sequencer using a third generation sequencing technology.

Optionally, the determining a target sequencer according to a sample property of the to-be-analyzed sample, the analysis demand of the to-be-analyzed sample, and the idle conditions of all the current sequencers includes: inputting the sample property of the to-be-analyzed sample and the analysis demand of the to-be-analyzed sample to a pre-configured target classification model to obtain a sequencer type, the sequencer type being outputted by the target classification model and applicable to the to-be-analyzed sample; where the target classification model is obtained by training with historical analysis samples including sample properties and analysis demands as training samples and sequencer types applicable to the training samples as sample labels; and determining, according to the sequencer type applicable to the to-be-analyzed sample and the idle conditions of all the current sequencers, the target sequencer.

Optionally, a training process for the target classification model includes: acquiring a plurality of historical analysis samples including sample properties and analysis demands and real sequencer types applicable to the plurality of the historical analysis samples, and dividing the plurality of historical analysis samples into a training set and a test set; inputting the historical analysis samples in the training set to an initial classification model to obtain predicted sequencer types, outputted by the initial classification model, of the historical analysis samples; training the initial classification model with a goal that the predicted sequencer types approach to the real sequencer types applicable to the historical analysis samples; and when the initial classification model meets a preset training condition, adjusting and optimizing the trained initial classification model by using the historical analysis samples in the test set to obtain the final target classification model.

Optionally, the determining a target sequencer according to a sample property of the to-be-analyzed sample, the analysis demand of the to-be-analyzed sample, the idle conditions of all the current sequencers includes: comparing the sample property of the to-be-analyzed sample and the analysis demand of the to-be-analyzed sample with corresponding thresholds respectively, or comparing the analysis demand of the to-be-analyzed sample with a corresponding threshold alone; and determining, according to a comparing result and the idle conditions of all the current sequencers, the target sequencer.

Optionally, the comparing the sample property of the to-be-analyzed sample and the analysis demand of the to-be-analyzed sample with corresponding thresholds respectively or comparing the analysis demand of the to-be-analyzed sample with a corresponding threshold alone, and determining, according to a comparing result and the idle conditions of all the current sequencers, the target sequencer includes: if the analysis demand of the to-be-analyzed sample includes an unlimited sequencing cost, a sequencing cost lower than a preset cost threshold, an analysis precision higher than a preset precision threshold, or a specific function detection requirement, taking both the first sequencer and the second sequencer as the target sequencer, and verifying a sequencing result from the target sequencer by means of a sequencer using a first generation sequencing technology; if the analysis demand of the to-be-analyzed sample includes the sequencing cost lower than the preset cost threshold, and an analysis accuracy higher than a preset accuracy threshold, determining, according to idle conditions of the first sequencers of different types, the target sequencer; if the analysis demand of the to-be-analyzed sample includes the sequencing cost lower than the preset cost threshold and the analysis accuracy higher than the preset accuracy threshold, and the sample property of the to-be-analyzed sample includes a raw data volume smaller than a preset data volume threshold, selecting a sequencer with a higher accuracy and a function meeting requirements as the target sequencer according to accuracies of the first sequencer and the second sequencer and whether functions of the first sequencer and the second sequencer meet requirements, or determining, according to idle conditions of the first sequencer and the second sequencer, the target sequencer in the case that the first sequencer and the second sequencer have the same accuracies and functions; and if the analysis demand of the to-be-analyzed sample includes the unlimited sequencing cost, the analysis precision lower than the preset precision threshold and a sequencing duration shorter than a preset sequencing duration, and an analysis environment where the to-be-analyzed sample is located is a non-normal environment, selecting Nanopore in the second sequencer as the target sequencer.

Optionally, the sequencing the to-be-analyzed sample by means of the target sequencer to obtain a base sequence corresponding to the to-be-analyzed sample includes: sequencing the to-be-analyzed sample by means of the target sequencer, and converting a sequencing result from the target sequencer into a binary sequence according to a sequencing mode of the target sequencer, to obtain the base sequence corresponding to the to-be-analyzed sample.

Optionally, the sequencing the to-be-analyzed sample by means of the target sequencer and converting a sequencing result from the target sequencer into a binary sequence according to a sequencing mode of the target sequencer to obtain the base sequence corresponding to the to-be-analyzed sample includes: if the target sequencer is the first sequencer, directly sequencing the to-be-analyzed sample by means of the first sequencer, performing, by means of an optical device, base recognition on a fluorescence signal obtained by sequencing, and converting a base recognition result into a binary sequence in a binary coded format to obtain the base sequence corresponding to the to-be-analyzed sample; or sequencing the to-be-analyzed sample by means of the first sequencer, and performing, by means of a semiconductor chip, light intensity value detection on a fluorescence signal obtained by sequencing, and converting a light intensity signal into an electric signal in a binary coded format to obtain the base sequence corresponding to the to-be-analyzed sample; if the target sequencer is the second sequencer, directly sequencing the to-be-analyzed sample by means of the second sequencer, and converting a current signal obtained by sequencing into a binary sequence in a binary coded format to obtain the base sequence corresponding to the to-be-analyzed sample; or sequencing the to-be-analyzed sample by means of the second sequencer, performing, by means of a semiconductor chip, light intensity value detection on a fluorescence signal obtained by sequencing, and converting a light intensity signal into an electric signal in a binary coded format to obtain the base sequence corresponding to the to-be-analyzed sample.

Optionally, the optical device includes a spectroscope and convex lenses; the performing, by means of an optical device, base recognition on a fluorescence signal obtained by sequencing and converting a base recognition result into a binary sequence in a binary coded format includes: dividing, by means of the spectroscope, a beam of photons corresponding to the fluorescence signal obtained by sequencing into four beams, filtering the four beams, and converting the four filtered beams into parallel light by means of four convex lenses; and converting the parallel light into a binary sequence by means of a CMOS camera.

Optionally, the performing, by means of a semiconductor chip, light intensity value detection on a fluorescence signal obtained by sequencing and converting a light intensity signal into an electric signal in a binary coded format includes: performing stacked packaging on an upper layer of the semiconductor chip, and performing, by means of the packaged semiconductor chip, intelligent color filter processing on the fluorescence signal obtained by sequencing, to allow the fluorescence signal color-filtered by four colors of light filters in an intelligent color filter to fall on a specified position of each of light filters in a lower light guide pipe; collecting the color-filtered fluorescence signal at the lower light guide pipe, and detecting a light intensity value of the color-filtered fluorescence signal by means of a photodiode in the semiconductor chip to obtain a detection result; and converting the detection result into the electric signal in the binary coded format by means of the CMOS camera.

Optionally, the invoking an analysis mode corresponding to a species to which the to-be-analyzed sample belongs and a sequencing technology used by the target sequencer to analyze the base sequence includes: if the species to which the to-be-analyzed sample belongs is a human having a reference genome, invoking a first analysis mode to analyze the base sequence when the target sequencer is the first sequencer, invoking a second analysis mode to analyze the base sequence when the target sequencer is the second sequencer, and invoking a third analysis mode to analyze the base sequence when the target sequencer is the first sequencer and the second sequencer; and if the species to which the to-be-analyzed sample belongs is other species not having a reference genome, invoking a first assembling mode to assemble the base sequence when the target sequencer is the first sequencer, invoking a second assembling mode to assemble the base sequence when the target sequencer is the second sequencer, and invoking a third assembling mode to assemble the base sequence when the target sequencer is the first sequencer and the second sequencer, and analyzing the assembled base sequence.

Optionally, the invoking a second analysis mode to analyze the base sequence when the target sequencer is the second sequencer includes: when the target sequencer is the second sequencer, performing sequence alignment, sorting, deduplication, variation detection, sequence annotation and interpretation on the base sequence to obtain the sequencing report; or performing sequence splicing on the base sequence by using a preset sequence splicing algorithm to obtain variation information corresponding to the base sequence, and performing sequence annotation and interpretation on the variation information to obtain the sequencing report.

Optionally, the invoking a third assembling mode to assemble the base sequence when the target sequencer is the first sequencer and the second sequencer and analyzing the assembled base sequence includes: when the target sequencer is the first sequencer and the second sequencer, determining evaluation results obtained by performing evaluation by a user on a plurality of assembling modes provided in advance according to different evaluation indexes, and selecting, according to the evaluation results, at least one assembling mode from the plurality of assembling modes provided in advance as the third assembling mode; and invoking the third assembling mode to assemble the base sequence, and analyzing the assembled base sequence.

Optionally, the invoking the third assembling mode to assemble the base sequence and analyzing the assembled base sequence includes: invoking the third assembling mode to assemble a base sequence of the first sequencer with a kmer cutting and de bruijn graph assembling method, and splicing the assembled base sequence with a base sequence of the second sequencer to obtain a spliced genome and corresponding variation information; or invoking the third assembling mode to align a base sequence of the first sequencer to a base sequence of the second sequencer with a kmer cutting and Hash alignment method, and after alignment data is obtained, performing error correction on the base sequence of the second sequencer according to the alignment data, and splicing the corrected base sequence with the base sequence of the first sequencer to obtain a spliced genome and corresponding variation information; or invoking the third assembling mode to splice a base sequence of the first sequencer into contig, performing error correction on a base sequence of the second sequencer by means of contig, and assembling the corrected base sequence to obtain a spliced genome and corresponding variation information; or invoking the third assembling mode to assemble a base sequence of the first sequencer with a kmer cutting and de bruijn graph assembling method, aligning a base sequence of the second sequencer to an assembled de bruijn graph, and constructing scaffold according to an alignment result to obtain a spliced genome and corresponding variation information.

Optionally, the invoking an analysis mode corresponding to a species to which the to-be-analyzed sample belongs and a sequencing technology used by the target sequencer to analyze the base sequence further includes: if the target sequencer is the first sequencer or the second sequencer, invoking a species tree construction mode corresponding to the species to which the to-be-analyzed sample belongs to construct a species tree corresponding to the species to which the to-be-analyzed sample belongs, and analyzing the base sequence according to the constructed species tree; or if the target sequencer is the first sequencer and the second sequencer, transcribing a base sequence of the first sequencer and a base sequence of the second sequencer to form transcriptome data, and performing a data alignment process and a data length, quality and integrity analysis process on the transcriptome data, to parse a regulation mechanism for metabolic expression of the species to which the to-be-analyzed sample belongs.

Optionally, the method further includes, when the target sequencer is the first sequencer and the second sequencer: if quality values of parts of loci in the base sequence or the assembled base sequence are lower than a preset quality threshold and directly affect an annotation result when a variation result of the base sequence is annotated or species annotation is performed on the assembled base sequence, verifying the parts of loci in a locus mutation manner or a short fragment mutation manner by means of a sequencer using a first generation sequencing technology.

The present disclosure further provides a flow sequencing and analysis apparatus. The apparatus includes: a data acquisition module configured to acquire a to-be-analyzed sample, an analysis demand of the to-be-analyzed sample, and idle conditions of all current sequencers; a sequencing module configured to determine a target sequencer according to a sample property of the to-be-analyzed sample, and the analysis demand of the to-be-analyzed sample, and the idle conditions of all the current sequencers, and sequence the to-be-analyzed sample by means of the target sequencer to obtain a base sequence corresponding to the to-be-analyzed sample, where the base sequence is a binary sequence; and an analysis module configured to input the base sequence to an internal storage, and invoke an analysis mode corresponding to a species to which the to-be-analyzed sample belongs and a sequencing technology used by the target sequencer to analyze the base sequence and generate a sequencing report.

The present disclosure further provides a storage medium. The storage medium has computer-readable instructions stored thereon. The computer-readable instructions, when executed by one or more processors, cause the one or more processors to perform the steps of the flow sequencing and analysis method according to any one of the above-mentioned embodiments.

The present disclosure further provides a computer device. The computer device includes one or more processors and a memory. The memory has computer-readable instructions stored thereon. The computer-readable instructions, when executed by the one or more processors, implement the steps of the flow sequencing and analysis method according to any one of the above-mentioned embodiments.

It can be seen from the above-mentioned technical solutions that the embodiments of the present disclosure have the following advantages.

According to the flow sequencing and analysis method and apparatus, the storage medium and the computer device provided in the present disclosure, after the to-be-analyzed sample and the corresponding analysis demand thereof and the idle conditions of all the current sequencers are acquired, the first sequencer using the second generation sequencing technology and the second sequencer using the third generation sequencing technology have different advantages, and therefore, in the present disclosure, the target sequencer can be determined in conjunction with the sample property of the to-be-analyzed sample and the analysis demand of the to-be-analyzed sample and the idle conditions of all the current sequencers. The target sequencer can be the first sequencer or the second sequencer, or can use the first sequencer and the second sequencer for mixed sequencing. In this way, not only diversified use scenarios can be provided for users, but also the sequencing efficiency and the sequencing precision can be improved to a certain extent. Next, in the present disclosure, the to-be-analyzed sample can be sequenced by means of the target sequencer to obtain the base sequence corresponding to the to-be-analyzed sample, the base sequence is the binary sequence, that is, an ACGT base can be stored and transmitted to the internal storage by means of a binary sequence of 00, 01, 10 and 11. Then, the analysis mode corresponding to the species to which the to-be-analyzed sample belongs and the sequencing technology used by the target sequencer is directly invoked in the internal storage to analyze the base sequence and generate the sequencing report. In this way, a basecall process of sequencing and a subsequent analysis process can be linked through the internal storage, and sequencing and analysis processes of the to-be-analyzed sample are implemented by one process, so that it is simple, convenient and fast for operating staff to operate. With the linkage based on the internal storage, the analysis efficiency can also be increased, and I/O congestion can be reduced. Moreover, the base sequence in the binary coded format shortens the read and write time, more facilitates subsequent analysis process, increases the analysis efficiency of the overall process and is strong in adaptability. By means of a novel coding mode and processing in the case that existing machine parameters are basically unchanged, the service life of a machine is prolonged while the program operation efficiency is increased without affecting the sequencing and analysis accuracy.

### BRIEF DESCRIPTION OF DRAWINGS

In order to describe the technical solutions in the embodiments of the present disclosure or the conventional art more clearly, the accompanying drawings required for describing the embodiments or the conventional art will be briefly introduced below. Apparently, the accompanying drawings in the following description show only some embodiments of the present disclosure, and those of ordinary skill in the art can still derive other accompanying drawings from these accompanying drawings without creative efforts.
FIG. 1 is a schematic flow diagram of a flow sequencing and analysis method provided in an embodiment of the present disclosure;
FIG. 2 is a schematic diagram showing comparison of sequencing properties, advantages and disadvantages corresponding to sequencers using different sequencing technologies provided in an embodiment of the present disclosure;
FIG. 3 is a schematic diagram showing an analysis process for a base sequence provided in an embodiment of the present disclosure;
FIG. 4 is a schematic structural diagram of a flow sequencing and analysis apparatus provided an embodiment of the present disclosure; and
FIG. 5 is a schematic diagram showing an internal structure of a computer device provided an embodiment of the present disclosure.

### DESCRIPTION OF EMBODIMENTS

Technical solutions in the embodiments of the present disclosure will be described clearly and completely below in conjunction with the accompanying drawings in the embodiments of the present disclosure. Apparently, the described embodiments are only a part of the embodiments of the present disclosure, not all the embodiments. Based on the embodiments in the present disclosure, all other embodiments obtained by those of ordinary skill in the art without creative efforts shall fall within the protection scope of the present disclosure.

At present, due to a huge gene data volume, a relatively single use scenario and high dependence on a server in an analysis process, when a large number of samples are inputted in parallel, it is easy to cause I/O congestion, and a thread or process may be suspended, which greatly affects the analysis efficiency and the analysis precision. Based on this, the present disclosure provides the following technical solutions which specifically refer to the following description.

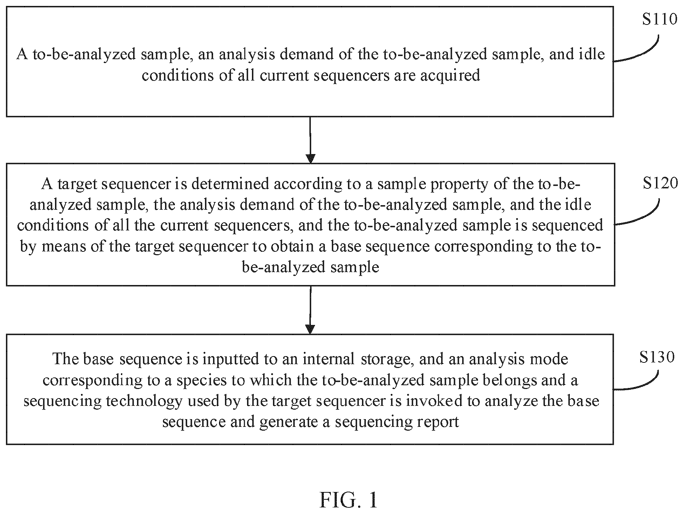

In an embodiment, as shown in FIG. 1 which is a schematic flow diagram of a flow sequencing and analysis method provided in an embodiment of the present disclosure, the present disclosure provides a flow sequencing and analysis method. The method includes steps S110 to S130.

In step S110, a to-be-analyzed sample, an analysis demand of the to-be-analyzed sample, and idle conditions of all current sequencers are acquired.

In the present step, during sequencing and analysis, the to-be-analyzed sample and the corresponding analysis demand thereof and the idle conditions of all the current sequencers can be acquired firstly. In this way, a target sequencer can be determined according to the to-be-analyzed sample and the corresponding analysis demand thereof and the idle conditions of all the current sequencers.

It can be understood that the to-be-analyzed sample in the present disclosure can be one sample or an item composed of a plurality of samples, which is not limited herein. In the present disclosure, the analysis demand of the to-be-analyzed sample refers to user' requirements for analysis precision, sequencing cost, sequencing and analysis emphasis or the like. In the present disclosure, all the current sequencers refer to a first sequencer using a second generation sequencing technology and a second sequencer using a third generation sequencing technology. The idle conditions of all the current sequencers refer to whether each of the current sequencers is working, whether there are still remaining resources to process the to-be-analyzed sample, or whether sequencing and analysis are performed when the to-be-analyzed sample needs to be sequenced and analyzed.

A work process of the second generation sequencing technology in the present disclosure mainly includes the following steps:
1) firstly, biochemical amplification and imaging are performed, and an img file is outputted: a to-be-tested DNA sequence is randomly interrupted and is connected by a connector for amplification (illumina is a bridge PCR+reversible termination method, i.e., 3', the uses a method for replicating a cssDNA library by a rolling circle, amplifying DNB nanospheres, and then, loading the DNA nanospheres onto a high-density grid), a biochemical reaction is performed, fluorescence is emitted, and photographing is performed to obtain an img file and the img file is outputted;
2) basecall: the img file is inputted, and a call file is outputted;
3) writefq: the call file is inputted, and a fq file is outputted;
4) data quality control: the fq file, a sample and online information are inputted, and a quality control result is outputted;
5) data archiving: fq is dually backed up and is archived; and
6) information analysis for NGS raw gene data: due to the different sizes and properties of raw data FASTQ of different brands and models of sequencers, different customized operations, such as separate splitting, merging and quality control of different parameters, are sometimes needed before processing, and then, bioinformatics analysis and interpretation are performed respectively.

The third generation sequencing technology in the present disclosure includes following two categories. One category is the application of single molecule real-time sequencing (SMRT), during sequencing, fluorescence is labeled on a phosphate group of deoxyribonucleotide, fluorescence-labeled nucleotides may send a fluorescence signal on a doping position, next, a DNA polymerase is immobilized at the bottom of a nano-chamber by using an insoluble enzyme, and the fluorescence signal is observed via micropores of 20-30 nm in the bottom of the nano-chamber by adopting a zero-mode waveguide technology (ZMW). The other one category is a third generation sequencer for sequencing based on biological nanopores, the DNA sequencing speed is reduced by means of molecular motors, process enzymes and ratchet effects on the nanopores, bases are distinguished by limiting loci, when a nucleic acid sequence passes, the conductivity of an ion current in the pores may change, the nucleotides have different shapes, and thus, the bases can be recognized according to influences of different nucleotides on the change of the ion current.

In step S120, a target sequencer is determined according to a sample property of the to-be-analyzed sample, the analysis demand of the to-be-analyzed sample, and the idle conditions of all the current sequencers, and the to-be-analyzed sample is sequenced by means of the target sequencer to obtain a base sequence corresponding to the to-be-analyzed sample.

In the present step, after the to-be-analyzed sample and the corresponding analysis demand thereof and the idle conditions of all the current sequencers are acquired in step S110, in the present disclosure, the target sequencer can be determined according to the sample property of the to-be-analyzed sample, the analysis demand of the to-be-analyzed sample, and the idle conditions of all the current sequencers. Next, the to-be-analyzed sample is sequenced by means of the target sequencer to obtain the base sequence corresponding to the to-be-analyzed sample.

It can be understood that sequencers using different sequencing technologies have different advantages and disadvantages, and different to-be-analyzed samples have different sample properties and analysis demands. Therefore, in order to obtain a more accurate sequencing result and meet demands of users, in the present disclosure, the target sequencer can be determined according to the sample property of the to-be-analyzed sample, the analysis demand of the to-be-analyzed sample, and the idle conditions of all the current sequencers, and thus, the sequencing efficiency and the sequencing precision are improved.

For example, a sequencing cost, a read length and a throughput are three important indexes for evaluating whether a sequencing technology is advanced or not. A first generation sequencing technology and a second generation sequencing technology are different in throughout and cost and are the same in core sequencing principle (except Solid based on a concept of sequencing while connecting) based on a concept of sequencing while synthesizing. The second generation sequencing technology has the advantages of greatly reducing the cost and greatly increasing the throughout as comparison with the first generation sequencing technology, and has the disadvantages of increasing the sequencing error rate to a certain extent duo to PCR introduction, the disadvantages of having system bias and having a relatively small read length. A third generation sequencing technology is developed for overcoming the disadvantages existing in the second generation sequencing technology, and has the fundamental characteristic of single molecule sequencing, which does not require any PCR process, thereby effectively avoiding system errors caused by PCR bias, increasing the read length, and maintaining the advantages of high throughput and low cost of the second generation sequencing technology.

For the to-be-analyzed sample in the present disclosure, the sample properties are diverse, raw data volumes of some samples are too large, and therefore, when sequencing and analysis are performed by using a sequencer with a higher sequencing cost, a high sequencing cost may be required. Raw data volumes of some samples are smaller or moderate, it should be considered whether there are requirements for analysis precision, and if there are requirements for the analysis precision, an appropriate sequencer needs to be selected according to specific analysis precision requirements. In addition, when any one sequencer can be selected for sequencing, it should also be considered whether each of the sequencers is idle, and the sequencer can be selected for sequencing and analysis only when the sequencer is idle.

In addition, in the present disclosure, the base sequence, obtained after the target sequencer is determined and the to-be-analyzed sample is sequenced by means of the target sequencer, is a binary sequence which not only can shorten the read and write time, and more facilitate the subsequent analysis process, but also increases the analysis efficiency of the overall process and is strong in adaptability. By means of a novel coding mode and processing in the case that existing machine parameters are basically unchanged, the service life of a machine can be prolonged while the program operation efficiency is increased without affecting the sequencing and analysis accuracy.

In step S130, the base sequence is inputted to an internal storage, and an analysis mode corresponding to a species to which the to-be-analyzed sample belongs and a sequencing technology used by the target sequencer is invoked to analyze the base sequence and generate a sequencing report.

In the present step, after step S120 that the target sequencer is determined and the to-be-analyzed sample is sequenced by means of the target sequencer to obtain the base sequence corresponding to the to-be-analyzed sample, in the present disclosure, the base sequence can be inputted to the internal storage, and the analysis mode corresponding to the species to which the to-be-analyzed sample belongs and the sequencing technology used by the target sequencer is invoked to analyze the base sequence, thereby generating the sequencing report.

Specifically, in the present disclosure, the base sequence in the binary coded format is transmitted to the internal storage, and then, the analysis mode corresponding to the species to which the to-be-analyzed sample belongs and the sequencing technology used by the target sequencer is directly invoked in the internal storage to analyze the base sequence and generate the sequencing report. In this way, a basecall process of sequencing and a subsequent analysis process can be linked through the internal storage, and sequencing and analysis processes of the to-be-analyzed sample are implemented by one process, so that it is simple, convenient and fast for operating staff to operate. With the linkage based on the internal storage, the analysis efficiency can also be increased, and I/O congestion can be reduced.

Further, in the present disclosure, when the base sequence is analyzed, both the species to which the to-be-analyzed sample belongs and the sequencing technology used by the target sequencer are considered. For example, when the to-be-analyzed sample is a human sample, there is a certain difference between analysis steps and specific detection contents of the human sample and analysis steps and detection contents of other species. For this difference, in the present disclosure, different analysis modes can be preset. For different sequencing technologies, advantages and disadvantages thereof are also different. In the present disclosure, the corresponding analysis mode can be preset by learning from each other or fostering strengths and circumventing weaknesses, in this way, the base sequence can be analyzed in various analysis modes, and then, the analysis precision of the base sequence can be improved while use scenarios are enriched.

In addition, in the present disclosure, when the sequencing report is generated, the sequencing report can be automatically generated according to an appointed report generation rule and/or a template invoking rule, thereby the analysis efficiency is increased while demands of users are met.

In the above-mentioned embodiment, after the to-be-analyzed sample and the corresponding analysis demand thereof and the idle conditions of all the current sequencers are acquired, the first sequencer using the second generation sequencing technology and the second sequencer using the third generation sequencing technology have different advantages, and therefore, in the present disclosure, the target sequencer can be determined in conjunction with the sample property of the to-be-analyzed sample and the analysis demand of the to-be-analyzed sample and the idle conditions of all the current sequencers. The target sequencer can be the first sequencer or the second sequencer, or can use the first sequencer and the second sequencer for mixed sequencing. In this way, not only diversified use scenarios can be provided for users, but also the sequencing efficiency and the sequencing precision can be improved to a certain extent. Next, in the present disclosure, the to-be-analyzed sample can be sequenced by means of the target sequencer to obtain the base sequence corresponding to the to-be-analyzed sample, the base sequence is the binary sequence, that is, an ACGT base can be stored and transmitted to the internal storage by means of a binary sequence of 00, 01, 10 and 11. Then, the analysis mode corresponding to the species to which the to-be-analyzed sample belongs and the sequencing technology used by the target sequencer is directly invoked in the internal storage to analyze the base sequence and generate the sequencing report. In this way, a basecall process of sequencing and a subsequent analysis process can be linked through the internal storage, and sequencing and analysis processes of the to-be-analyzed sample are implemented by one process, so that it is simple, convenient and fast for operating staff to operate. With the linkage based on the internal storage, the analysis efficiency can also be increased, and I/O congestion can be reduced. Moreover, the base sequence in the binary coded format shortens the read and write time, more facilitates subsequent analysis process, increases the analysis efficiency of the overall process and is strong in adaptability. By means of a novel coding mode and processing in the case that existing machine parameters are basically unchanged, the service life of a machine is prolonged while the program operation efficiency is increased without affecting the sequencing and analysis accuracy.

In an embodiment, that a target sequencer is determined according to a sample property of the to-be-analyzed sample, the analysis demand of the to-be-analyzed sample, and the idle conditions of all the current sequencers in step S120 can include following steps.

In step S101, the sample property of the to-be-analyzed sample and the analysis demand of the to-be-analyzed sample are inputted to a pre-configured target classification model to obtain a sequencer type, the sequencer type being outputted by the target classification model and applicable to the to-be-analyzed sample. The target classification model is obtained by training with historical analysis samples including sample properties and analysis demands as training samples and sequencer types applicable to the training samples as sample labels.

In step S102, the target sequencer is determined according to the sequencer type applicable to the to-be-analyzed sample and the idle conditions of all the current sequencers.

In the present embodiment, when the target sequencer is determined, in the present disclosure, the pre-configured target classification model can be used. The target classification model is obtained by training with the historical analysis samples including the sample properties and the analysis demands as the training samples and the sequencer types applicable to the training samples as the sample labels. Therefore, in the present disclosure, the sample property of the to-be-analyzed sample and the analysis demand of the to-be-analyzed sample can be inputted to the target classification model, and the target classification model can output the sequencer type corresponding to the to-be-analyzed sample. Next, in the present disclosure, the final target sequencer can be determined in conjunction with the sequencer type outputted by the target classification model and the idle conditions of all the current sequencers.

In the present disclosure, the sample property of the to-be-analyzed sample and the analysis demand of the to-be-analyzed sample include, but are not limited to a sample size, a sequencing type, a sequencing depth, a sequencing duration, sequencing cost, and sequencing accuracy. In the present disclosure, corresponding functions can be constructed on the basis of the sample properties and the analysis demands of all the historical analysis samples, for example, the sequencer type=f(a sample size, a sequencing type, a sequencing depth, a sequencing duration, sequencing cost, and sequencing accuracy), where f represents a function and can be obtained by modeling and training existing data. Specifically, in the present disclosure, a classification model can be trained by adopting a classification algorithm (fusion of a decision tree model, a support vector machine model, a neural network model, and other models) in machine learning to predict a sequencer type applicable to current conditions.

After the sequencer type is obtained by means of the target classification model, in the present disclosure, the final target sequencer also needs to be determined according to the idle conditions of all the current sequencer. In this way, not only can the sequencing waiting time period be shortened, but also the sequencing efficiency can be increased.

In an embodiment, a training process for the target classification model in step S101 can include following steps.

In step S1010, a plurality of historical analysis samples including sample properties and analysis demands and real sequencer types applicable to the plurality of the historical analysis samples are acquired, and the plurality of historical analysis samples are divided into a training set and a test set.

In step S1011, the historical analysis samples in the training set are inputted to an initial classification model to obtain predicted sequencer types, outputted by the initial classification model, of the historical analysis samples.

In step S1012, the initial classification model is trained with a goal that the predicted sequencer types approach to the real sequencer types applicable to the historical analysis samples.

In step S1013, when the initial classification model meets a preset training condition, the trained initial classification model is adjusted and optimized by using the historical analysis samples in the test set to obtain the final target classification model.

In the present embodiment, when the target classification model is trained, the plurality of historical analysis samples including the sample properties and the analysis demands and the real sequencer types applicable to the historical analysis samples can be acquired firstly, and then, the plurality of historical analysis samples can be divided into the training set and the test set, the historical analysis samples in the training set can be used for training the initial classification model, the historical analysis samples in the test set can be used for testing performances of the trained initial classification model, and the initial classification model is adjusted and optimized according to a test result, so that the accuracy and generalization ability of the model are improved.

Specifically, in the present disclosure, when the model is trained, a dataset including a plurality of historical analysis samples can be prepared according to past sequencing experience, each of the historical analysis samples includes a sample size, a sequencing type, a sequencing depth, a sequencing duration, sequencing cost, sequencing accuracy, and a corresponding sequencer type. Then, in the present disclosure, the dataset can be divided into a training set and a test set, the initial classification model is trained by using the training set, performances of the trained initial classification model are tested by using the test set, and the initial classification model is adjusted and optimized according to a test result to improve the accuracy and the generalization ability of the model, so that the final target classification model can be obtained.

In an embodiment, that a target sequencer is determined according to a sample property of the to-be-analyzed sample, the analysis demand of the to-be-analyzed sample, and the idle conditions of all the current sequencers in step S120 includes the following step.

In step S121, the sample property of the to-be-analyzed sample and the analysis demand of the to-be-analyzed sample are compared with corresponding thresholds respectively, or the analysis demand of the to-be-analyzed sample is compared with a corresponding threshold alone; and the target sequencer is determined according to a comparing result and the idle conditions of all the current sequencers.

In the present embodiment, when the target sequencer is determined according to the sample property of the to-be-analyzed sample, the analysis demand of the to-be-analyzed sample, and the idle conditions of all the current sequencers, in the present disclosure, the sample property of the to-be-analyzed sample and the analysis demand of the to-be-analyzed sample can be compared with the corresponding thresholds respectively, or the analysis demand of the to-be-analyzed sample is directly compared with the corresponding threshold alone, and next, the target sequencer is determined according to the comparing result and the idle conditions of all the current sequencers.

It should be noted that there may be a general condition and a specific condition in the sample property of the to-be-analyzed sample, for example, raw data volume is too large, or the raw data volume is too small, or the raw data volume is normal. When there is the specific condition in the sample property of the to-be-analyzed sample, in the present disclosure, the sample property of the to-be-analyzed sample and the analysis demand of the to-be-analyzed sample can be compared with the corresponding thresholds respectively. When the sample property of the to-be-analyzed sample is under the general condition, the analysis demand of the to-be-analyzed sample is only considered, and the target sequencer is determined according to the comparing result, between the analysis demand and the corresponding threshold, and the idle conditions of all the current sequencers.

In an embodiment, that the sample property of the to-be-analyzed sample and the analysis demand of the to-be-analyzed sample are compared with corresponding thresholds respectively or the analysis demand of the to-be-analyzed sample is compared with a corresponding threshold alone, and the target sequencer is determined according to a comparing result and the idle conditions of all the current sequencers in step S121 can include following steps.

In step S1210, if the analysis demand of the to-be-analyzed sample includes an unlimited sequencing cost, a sequencing cost lower than a preset cost threshold, an analysis precision higher than a preset precision threshold, or a specific function detection requirement, both the first sequencer and the second sequencer are taken as the target sequencer, and a sequencing result from the target sequencer is verified by means of a sequencer using a first generation sequencing technology.

In step S1211, if the analysis demand of the to-be-analyzed sample includes the sequencing cost lower than the preset cost threshold, and an analysis accuracy higher than a preset accuracy threshold, the target sequencer is determined according to idle conditions of the first sequencers of different types.

In step S1212, if the analysis demand of the to-be-analyzed sample includes the sequencing cost lower than the preset cost threshold and the analysis accuracy higher than the preset accuracy threshold, and the sample property of the to-be-analyzed sample includes a raw data volume smaller than a preset data volume threshold, a sequencer with a higher accuracy and a function meeting requirements is selected as the target sequencer according to accuracies of the first sequencer and the second sequencer and whether functions of the first sequencer and the second sequencer meet requirements, or the target sequencer is determined according to idle conditions of the first sequencer and the second sequencer in the case that the first sequencer and the second sequencer have the same accuracies and functions.

In step S1213, if the analysis demand of the to-be-analyzed sample includes the unlimited sequencing cost, the analysis precision lower than the preset precision threshold and a sequencing duration shorter than a preset sequencing duration, and an analysis environment where the to-be-analyzed sample is located is a non-normal environment, Nanopore in the second sequencer is selected as the target sequencer.

In the present embodiment, when the target sequencer is determined, the sample property of the to-be-analyzed sample and the analysis demand of the to-be-analyzed sample can be compared with the corresponding thresholds respectively, or the analysis demand of the to-be-analyzed sample is directly compared with the corresponding threshold alone, and the target sequencer is determined according to the comparing result and the idle conditions of all the current sequencers.

Specifically, in the present disclosure, the analysis demand of the to-be-analyzed sample can be compared with the corresponding threshold alone, for example, the sequencing cost is compared with the preset cost threshold, the analysis precision is compared with the preset precision threshold, the analysis accuracy is compared with the preset accuracy threshold, or the sequencing duration is compared with the preset sequencing duration. Alternatively, in the present disclosure, the sample property of the to-be-analyzed sample and the analysis demand of the to-be-analyzed sample can be compared with the corresponding thresholds respectively. The comparison for the analysis demand is consistent with the comparison as described above. With respect for the comparison for the sample property, the raw data volume of the to-be-analyzed sample can be compared with the preset data volume threshold in the present disclosure. Apparently, after the comparison, the analysis environment where the to-be-analyzed sample is located, whether the accuracies and functions of the sequencers meet the requirements and other conditions can also be introduced, which is not limited herein.

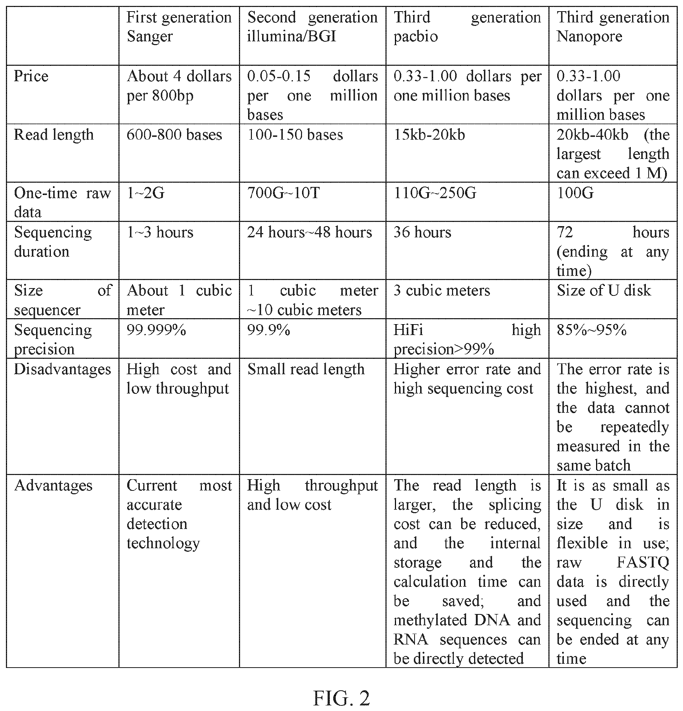

In a specific implementation, as shown in FIG. 2 which is a schematic diagram showing comparison of sequencing properties, advantages and disadvantages corresponding to sequencers using different sequencing technologies provided in an embodiment of the present disclosure, it can be seen from FIG. 2 that the first generation sequencing technology is applicable to few locus verification; the second generation sequencing technology is low in cost and high in throughput (the one-time raw data volume is high); and the third generation pacbio sequencing technology is high in cost, but has no obvious disadvantage in cost during detection of data in a small interval captured with a smaller data volume; and the third generation nanopore sequencing technology is high in cost, and requires a small sequencer, this detection can be used for pathogenic microbial infections (patients who has a fever of unknown origin) having no high precision requirements in remote areas, and the sequencer is convenient to carry.

Based on this, in the present disclosure, the target sequencer is determined according to the sample property of the to-be-analyzed sample, the analysis demand of the to-be-analyzed sample, and the idle conditions of all the current sequencers. Specifically, if the analysis demand of the to-be-analyzed sample includes the unlimited sequencing cost and a higher analysis precision, the first sequencer and the second sequencer can be used as the target sequencer, and the sequencing result from the target sequencer is verified by means of the sequencer using the first generation sequencing technology. If the analysis demand of the to-be-analyzed sample includes the sequencing cost lower than the preset cost threshold, a higher analysis precision, and the specific function detection requirement, such as detection for methylation, RNA, GC range, highly-variable area detection and pseudo genes are required, the first sequencer and the second sequencer can also be selected as the target sequencer, and the sequencing result from the target sequencer is verified by means of the sequencer using the first generation sequencing technology.

Further, if the analysis demand of the to-be-analyzed sample includes the sequencing cost lower than the preset cost threshold and the analysis accuracy higher than the preset accuracy threshold, the target sequencer can be determined according to the idle conditions of the different types of first sequencers, such as an illumina sequencer and a Beijing Genomics Institute sequencer in which the idle one is selected. If the analysis demand of the to-be-analyzed sample includes the sequencing cost lower than the preset cost threshold and the analysis accuracy higher than the preset accuracy threshold, and the sample property of the to-be-analyzed sample includes the raw data volume smaller than the preset data volume threshold, the sequencer with a higher accuracy and a function meeting requirements is selected as the target sequencer according to accuracies of the first sequencer and the second sequencer and whether functions of the first sequencer and the second sequencer meet requirements, or the target sequencer is determined according to idle conditions of the first sequencer and the second sequencer in the case that the first sequencer and the second sequencer have the same accuracies and functions. If the analysis demand of the to-be-analyzed sample includes the unlimited sequencing cost, a lower analysis precision and a higher sequencing duration, and the analysis environment where the to-be-analyzed sample is located is the non-normal environment, such as an area where the climate is relatively harsh, a relatively remote area, and an area where the traffic is inconvenient, in this case, the Nanopore in the second sequencer can be selected as the target sequencer which is convenient for operating staff to carry, and therefore, nearby treatment can be achieved.

The above-mentioned preset precision threshold refers to a precision threshold related to the analysis precision, such as overall analysis precision and analysis precision of each detection part, which is not limited herein.

In an embodiment, that the to-be-analyzed sample is sequenced by means of the target sequencer to obtain a base sequence corresponding to the to-be-analyzed sample in step S 120 can include the following step.

In step S210, the to-be-analyzed sample is sequenced by means of the target sequencer, and a sequencing result from the target sequencer is converted into a binary sequence according to a sequencing mode of the target sequencer to obtain the base sequence corresponding to the to-be-analyzed sample.

In the present embodiment, when the to-be-analyzed sample is sequenced by means of the target sequencer to obtain the base sequence corresponding to the to-be-analyzed sample, in the present disclosure, the sequencing result from the target sequencer can be converted into the binary sequence according to the sequencing mode of the target sequencer after the to-be-analyzed sample is sequenced by means of the target sequencer, thereby obtaining the base sequence corresponding to the to-be-analyzed sample.

For example, since different sequencers use different sequencing modes, such as the second generation sequencing technology or the third generation sequencing technology, there are also different technical branches under the second generation sequencing technology or the third generation sequencing technology, when the to-be-analyzed sample is sequenced by adopting different sequencing technologies, different sequencing results are also obtained. Therefore, in the present disclosure, corresponding sequence conversion modes can be set for target sequencers using different sequencing modes, and the sequencing result from the target sequencer can be converted into a binary sequence in the corresponding sequence conversion mode, and then, the base sequence corresponding to the to-be-analyzed sample is obtained.

In an embodiment, that the to-be-analyzed sample is sequenced by means of the target sequencer, and a sequencing result from the target sequencer is converted into a binary sequence according to a sequencing mode of the target sequencer to obtain the base sequence corresponding to the to-be-analyzed sample in step S210 can include following steps.

In step S211, if the target sequencer is the first sequencer, the to-be-analyzed sample is directly sequenced by means of the first sequencer, base recognition is performed, by means of an optical device, on a fluorescence signal obtained by sequencing, and a base recognition result is converted into a binary sequence in a binary coded format to obtain the base sequence corresponding to the to-be-analyzed sample.

In step S212, alternatively, the to-be-analyzed sample is sequenced by means of the first sequencer, and light intensity value detection is performed, by means of a semiconductor chip, on a fluorescence signal obtained by sequencing, and a light intensity signal is converted into an electric signal in a binary coded format to obtain the base sequence corresponding to the to-be-analyzed sample.

In step S213, if the target sequencer is the second sequencer, the to-be-analyzed sample is directly sequenced by means of the second sequencer, and a current signal obtained by sequencing is converted into a binary sequence in a binary coded format to obtain the base sequence corresponding to the to-be-analyzed sample.

In step S214, alternatively, the to-be-analyzed sample is sequenced by means of the second sequencer, light intensity value detection is performed, by means of a semiconductor chip, on a fluorescence signal obtained by sequencing, and a light intensity signal is converted into an electric signal in a binary coded format to obtain the base sequence corresponding to the to-be-analyzed sample.

In the present embodiment, if the target sequencer is the first sequencer, i.e., the sequencer using the second generation sequencing technology, the sequencer using the second generation sequencing technology performs the base recognition, by means of the optical device, on the fluorescence signal obtained by sequencing, and therefore, in the present disclosure, the sequencing result from the target sequencer can be converted into the binary sequence by changing the basecalling logic of a part of upstream second generation sequencers or directly changing a sequence format of the sequencing result without changing the basecalling logic thereof. If the target sequencer is the second sequencer, i.e., the sequencer using the third generation sequencing technology, the sequencer using the third generation sequencing technology is mainly divided into two categories: one is a sequencer for SMRT, and the other one is a sequencer for sequencing based on biological nanopores, and sequencing results generated by the two categories of sequencers are different. Therefore, for the different sequencing technologies, in the present disclosure, different conversion modes can also be used to convert the sequencing result from the target sequencer into the binary sequence.

In a specific implementation, if the target sequence is the first sequencer, the to-be-analyzed sample can be directly sequenced by means of the first sequencer, base recognition can be performed, by means of the optical device, on the fluorescence signal obtained by sequencing, then, the base recognition result is converted into the binary sequence by adopting the binary coded format, and thus, the base sequence corresponding to the to-be-analyzed sample is obtained. Alternatively, the to-be-analyzed sample is sequenced by means of the first sequencer, and the semiconductor chip is adopted to perform light intensity value detection on the fluorescence signal obtained by sequencing and then convert the light intensity signal into the electric signal in the binary coded format, and thus, the base sequence corresponding to the to-be-analyzed sample is obtained.

For the second sequencer using an SMRT technology, during sequencing, fluorescence is labeled on a phosphate group of deoxyribonucleotide, fluorescence-labeled nucleotides may send a fluorescence signal on a doping position, a DNA polymerase is immobilized at the bottom of a nano-chamber by PacBio by using an insoluble enzyme, and the fluorescence signal is observed via micropores of 20-30 nm in the bottom of the nano-chamber by adopting a zero-mode waveguide technology (ZMW), so that the signal-to-noise ratio and the accuracy are improved. Therefore, in the present disclosure, the to-be-analyzed sample can be sequenced by means of the second sequencer, and the semiconductor chip is adopted to perform light intensity value detection on the fluorescence signal obtained by sequencing and then convert the light intensity signal into the electric signal in the binary coded format, and thus, the base sequence corresponding to the to-be-analyzed sample is obtained. For the second sequencer for sequencing based on biological nanopores, the DNA sequencing speed is reduced by means of molecular motors, process enzymes and ratchet effects on the nanopores, bases are distinguished by limiting loci, when a nucleic acid sequence passes, the conductivity of an ion current in the pores may change, the nucleotides have different shapes, and thus, the bases can be recognized according to influences of different nucleotides on the change of the ion current. Therefore, in the present disclosure, the to-be-analyzed sample can also be sequenced by means of the second sequencer, and a current signal obtained by sequencing is converted into a binary sequence by adopting a binary coded format, and then, the base sequence corresponding to the to-be-analyzed sample is obtained.

In the above-mentioned embodiment, the sequencer using the second generation sequencing technology or the sequencer using the third generation sequencing technology can be used to put a fastq result outputted by basecall into the internal storage, or put the electric signal converted from a light intensity value detected after intelligently color-filtering is performed by the semiconductor chip, i.e., ACGT represented by 00, 01, 10 and 11, into the internal storage for subsequent alignment and assembly analysis. Twice network transmissions and I/O read and write are reduced in the overall process, so that the analysis efficiency is increased. Moreover, a Fastq format is not an existing format that four rows form a group any more, while a new format of 00/01/10/11 is adopted for representing the ATCG, in this way, the read and write analysis efficiency is also further increased. In addition, in the present disclosure, processes such as biochemical imaging, basecall or photoelectric signal conversion and alignment not requiring to output results are linked by the internal storage in sequencing and analysis processes, results can be outputted in a customized mode (new fastq is outputted or not), and therefore, the I/O communication can be further reduced.

In an embodiment, the optical device includes a spectroscope and convex lenses.

That base recognition is performed, by means of an optical device, on a fluorescence signal obtained by sequencing, and a base recognition result is converted into a binary sequence in a binary coded format in step S211 can include following steps.

In step S2110: a beam of photons corresponding to the fluorescence signal obtained by sequencing is divided into four beams by means of the spectroscope, the four beams are filtered, and the four filtered beams are converted into parallel light by means of the four convex lenses.

In step S2111: the parallel light is converted into a binary sequence by means of a CMOS camera.

In the present embodiment, when the base recognition is performed, by means of the optical device, on the fluorescence signal obtained by sequencing, firstly, the photons corresponding to the fluorescence signal obtained by sequencing can be divided into the four beams by means of the spectroscope, the four beams can be respectively filtered, then, the four filtered beams are converted into the parallel light by means of the corresponding number of convex lenses, and next, optical signals corresponding to the parallel light are converted into binary electric signal sequences, such as 00, 01, 10 and 11, by means of the CMOS element.

In an embodiment, that light intensity value detection is performed, by means of a semiconductor chip, on a fluorescence signal obtained by sequencing and a light intensity signal is converted into an electric signal in a binary coded format in step S212 or step S214 can include following steps.

In step S401, stacked packaging is performed on an upper layer of the semiconductor chip, and intelligent color filter processing is performed, by means of the packaged semiconductor chip, on the fluorescence signal obtained by sequencing, to allow the fluorescence signal color-filtered by four colors of light filters in an intelligent color filter to fall on a specified position of each of light filters in a lower light guide pipe.

In step S402, the color-filtered fluorescence signal is collected at the lower light guide pipe, and a light intensity value of the color-filtered fluorescence signal is detected by means of a photodiode in the semiconductor chip to obtain a detection result.

In step S403, the detection result is converted into the electric signal in the binary coded format by means of the CMOS camera.

In the present embodiment, when the target sequencer is the first sequencer or the second sequencer and the light intensity value detection is performed, by means of the semiconductor chip, on the fluorescence signal obtained by sequencing, in the present disclosure, the stacked packaging can be performed on the upper layer of the semiconductor chip firstly, then, the packaged semiconductor chip is used to perform the intelligent color filter processing on the fluorescence signal obtained by sequencing. The photons fall on specified positions of fluorescence colors (four light filters correspond to four positions) after passing through four colors of light filters, the fluorescence signal is collected at the lower light guide pipe, photon signal intensity detection is performed by the photodiode (trap) according to a light intensity value of fluorescence emitted by the semiconductor chip, and next, the optical signals are converted into electric signals by means of the CMOS camera. That is, a fluorescent sphere covers a transparent silicon wafer, the optical signals can be directly converted into the electric signals by means of the CMOS camera without convex lens concentration, and ACGT can be stored and transmitted by means of a binary sequence of 00, 01, 10 and 11. Compared with those of an optical image, more base sequences can be detected in a unit space, basecall and fastq file conversion events are avoided, the subsequent storage and analysis processes are optimized, and the problems of long time consumption, low analysis efficiency, complicated process and stronger dependence on storage, I/O and network performances are solved.

It can be understood that the semiconductor is an electronic component made of a semiconductor material, is usually a small-sized and integrated circuit, and is used for current control and amplification, operation, data storage, etc. The semiconductor chip can be used for fabricating various electronic devices, such as a computer, a mobile phone, a television, an air conditioner, and an automobile. Therefore, in the present disclosure, the semiconductor chip can be individually designed so as to have functions of performing the intelligent color filter processing on the fluorescence signal obtained by sequencing, collecting the color-filtered fluorescence signal at the lower light guide pipe, and detecting the light intensity value of the color-filtered fluorescence signal. In addition, in the present disclosure, the stacked packaging mode adopted by the semiconductor chip can be a packaging mode in the conventional art, which is not limited herein.

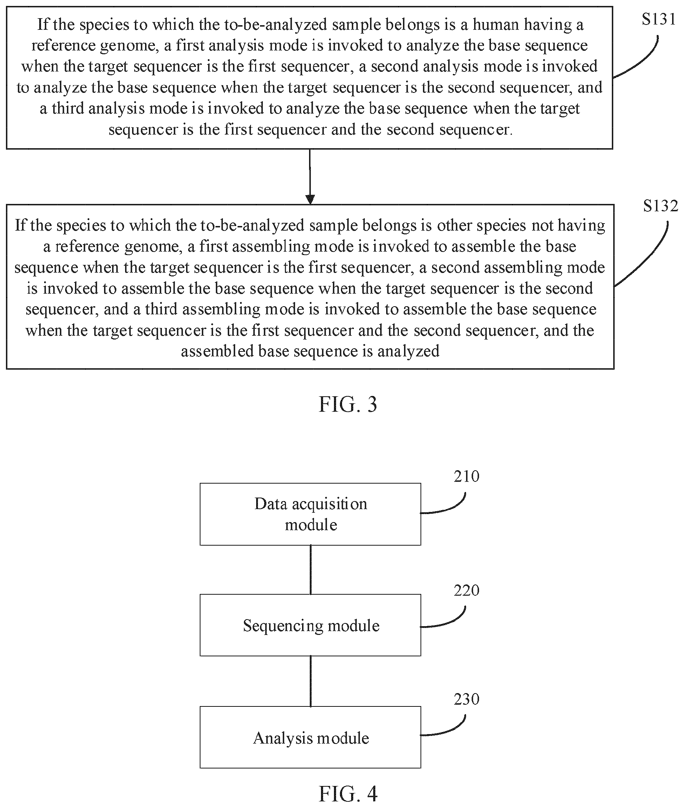

In an embodiment, as shown in FIG. 3 which is a schematic diagram showing an analysis process for a base sequence provided in an embodiment of the present disclosure, that an analysis mode corresponding to a species to which the to-be-analyzed sample belongs and a sequencing technology used by the target sequencer is invoked to analyze the base sequence in step S130 can include following steps.

In step S131, if the species to which the to-be-analyzed sample belongs is a human having a reference genome, a first analysis mode is invoked to analyze the base sequence when the target sequencer is the first sequencer, a second analysis mode is invoked to analyze the base sequence when the target sequencer is the second sequencer, and a third analysis mode is invoked to analyze the base sequence when the target sequencer is the first sequencer and the second sequencer.

In step S132, if the species to which the to-be-analyzed sample belongs is other species not having a reference genome, a first assembling mode is invoked to assemble the base sequence when the target sequencer is the first sequencer, a second assembling mode is invoked to assemble the base sequence when the target sequencer is the second sequencer, and a third assembling mode is invoked to assemble the base sequence when the target sequencer is the first sequencer and the second sequencer, and the assembled base sequence is analyzed.

In the present embodiment, since there is a certain difference in the species to which the to-be-analyzed samples belong, such as the human having the reference genome or the other species not having the reference genome. There is a certain difference in sequencing and analysis processes for different species. For example, when the human having the reference genome is analyzed in the present disclosure, analysis steps of the first sequencer or the second sequencer include processes of sequence alignment, sorting, deduplication, variation detection, and annotation. An analysis process for the other species not having the reference genome can include variation analysis for individual Snp, indel, cnv, sv, etc. based on the genome and a result/GC high-repetition area and low-complexity area analysis, or species evolution and genetic relationship construction, species annotation and abundance analysis, and research on correlation evolution between a phenome and typings thereof and each of a proteome, a metabolome, a methylation group, a transcriptome, etc. Therefore, for different species, different analysis modes can be adopted in the present disclosure. Apparently, the sequencing technology used by the target sequencer also needs to be considered, different sequencing technologies correspond to different sequencing modes, and therefore, there may also be a certain difference in subsequent analysis modes.

Specifically, if the species to which the to-be-analyzed sample belongs is the human having the reference genome, in the present disclosure, the first analysis mode can be invoked to analyze the base sequence when the target sequencer is the first sequencer, the second analysis mode can be invoked to analyze the base sequence when the target sequencer is the second sequencer, and the third analysis mode can be invoked to analyze the base sequence when the target sequencer is the first sequencer and the second sequencer. The first analysis mode in the present disclosure refers to a sequencing mode used by the sequencer using the second generation sequencing technology, for example, sequence alignment, sorting, deduplication, variation detection and annotation are performed according to a common mode. The second analysis mode in the present disclosure refers to a sequencing mode used by the sequencer using the third generation sequencing technology, for example, when sequence alignment, sorting, deduplication, variation detection and annotation are performed, an adaptive length alignment algorithm is automatically adopted according to different flow raw sequence lengths. The third analysis mode in the present disclosure refers to a process of performing sequence alignment, sorting, deduplication, variation detection and annotation in conjunction with advantages of the second generation sequencing technology and advantages of the third generation sequencing technology.

For example, when the second generation sequencing technology and the third generation sequencing technology are used in the present disclosure, variation detection can be performed according to the alignment results of the second generation sequencing technology and the third generation sequencing technology, and particularly in aspects, such as a repetition area, a high-GC-complexity area, a pseudo gene area, a methylation area, and a complex variation area, the third generation sequencing technology has advantages in detecting 1k-10k CNV areas or removing short repetition of STR. In a part with a lower detecting quality implemented by the third generation sequencing technology, a third generation large length result can be corrected by measuring more passes or according to data with a short read length and high precision detected by using the second generation sequencing technology, second generation reads are aligned to a third generation large length, base recognition and error correction are performed on a short sequence alignment result with the highest alignment value under a kmer sliding window, and the result covers a gap position to correct the large length, so that the most accurate mapping result is obtained, and then data redundancy is removed. Moreover, in the present disclosure, a software algorithm is selected from software algorithms including snp, indel, cnv and sv according to variation detection demands of different items, and variation detection results are obtained. The variation detection results can be used for transcript structural analysis, differential enrichment analysis, etc. In addition, in the present disclosure, during annotation, the variation result is comprehensively annotated by means of the annotation and filtering of bioinformatics analysis, basic information of variation under the combined action of the second generation sequencing technology or the third generation sequencing technology, disease information, a population frequency, software prediction results and pathogenicity reports, and pathogenicity variation is selected by comprehensively considering biological hazards, genetic anastomosis, and clinical feature anastomosis.

If the species to which the to-be-analyzed sample belongs is other species not having the reference genome, in the present disclosure, the first assembling mode can be invoked to assemble the base sequence when the target sequencer is the first sequencer, the second assembling mode can be invoked to assemble the base sequence when the target sequencer is the second sequencer, and the third assembling mode can be invoked to assemble the base sequence when the target sequencer is the first sequencer and the second sequencer, and the assembled base sequence can be analyzed.

In a specific implementation, in the present disclosure, denovo sequence assembly can be performed in conjunction with characteristics of the second generation sequencing technology and the third generation sequencing technology, and de novo assembly can be performed on a 50-400 bp sequence by the second generation sequencing by adopting an OLC or a de burin graph; de novo assembly is performed on data of which the length is about 1 k-40 kbp by the third generation sequencing or a graph, the OLC, or the like. When the second generation sequencing technology and the third generation sequencing technology are used for the combined action, base error correction can be performed according to a third generation sequencing result, a gap is repaired by means of a third generation assembled result. Next, subsequent species evolution and genetic relationship construction, species annotation and abundance analysis, large fragment CNV, GC high-repetition area and low-complexity area analysis, representation of subspecies, subpopulations and individual strains of the species, individual genome analysis (a relationship between a phenotype and each of specific sections, genes, and nucleotide variation) and integration of different omics (research on correlation evolution between a phenome and typings thereof and each of a proteome, a metabolome, a methylation group, a transcriptome, etc.) are performed, and thus, relevant results are obtained.

In an embodiment, that a second analysis mode is invoked to analyze the base sequence when the target sequencer is the second sequencer in step S131 can include following steps.

In step S1311, when the target sequencer is the second sequencer, sequence alignment, sorting, deduplication, variation detection, sequence annotation and interpretation are performed on the base sequence to obtain the sequencing report.

In step S1312, alternatively, sequence splicing is performed on the base sequence by using a preset sequence splicing algorithm to obtain variation information corresponding to the base sequence, and sequence annotation and interpretation are performed on the variation information to obtain the sequencing report.

In the present embodiment, when the target sequencer is the second sequencer, i.e., the sequencer using the third generation sequencing technology, in the present disclosure, the second analysis mode can be used to analyze the base sequence.

Specifically, the second analysis mode in the present disclosure can be specifically that the sequence alignment, the sorting, the deduplication, the variation detection, the sequence annotation and the interpretation are performed on the base sequence to obtain the sequencing report; alternatively, the sequence splicing is performed on the base sequence by using the preset sequence splicing algorithm to obtain the variation information corresponding to the base sequence, and next, the sequence annotation and the interpretation are performed on the variation information to obtain the sequencing report.

In the present disclosure, the sequence splicing can be performed on the base sequence by presetting a plurality of sequence splicing algorithms, such as a greedy algorithm, an OLC algorithm, and a De Bruijn graph method, which can be specifically selected according to actual conditions and is not limited herein.

In an embodiment, that when the target sequencer is the first sequencer and the second sequencer, a third assembling mode is invoked to assemble the base sequence, and the assembled base sequence is analyzed in step S132 can include following steps.

In step S1321, when the target sequencer is the first sequencer and the second sequencer, evaluation results obtained by performing evaluation by a user on a plurality of assembling modes provided in advance according to different evaluation indexes are determined, and at least one assembling mode is selected, according to the evaluation results, from the plurality of assembling modes provided in advance as the third assembling mode.

In step S1322, the third assembling mode is invoked to assemble the base sequence, and the assembled base sequence is analyzed.

In the present embodiment, when the first sequencer and the second sequencer are used for performing mixed sequencing on other species not having the reference genome, the user can evaluate, according to the different evaluation indexes, the plurality of assembling modes provided in advance and obtain the corresponding evaluation results. In this case, at least one assembling mode is selected, according to the evaluation results, from the plurality of assembling modes provided in advance as the third assembling mode. The third assembling mode is invoked to assemble the base sequence, and then, the assembled base sequence is analyzed.

When the user evaluates, according to the different evaluation indexes, the plurality of assembling modes provided in advance, the evaluation results include, but are not limited to a splicing length, an error rate, operation time, and a genome completion length. When the different assembling modes are evaluated, comprehensive evaluation for multi-dimensional, such as analysis performances, accuracies, advantages and disadvantages, can also be performed on the different assembling modes, which can be specifically set according to actual conditions so as not to be limited herein.

In an embodiment, that the third assembling mode is invoked to assemble the base sequence and the assembled base sequence is analyzed in step S1322 can include following steps.

In step S3221, the third assembling mode is invoked to assemble a base sequence of the first sequencer with a kmer cutting and de bruijn graph assembling method, and the assembled base sequence is spliced with a base sequence of the second sequencer to obtain a spliced genome and corresponding variation information.

In step S3222, alternatively, the third assembling mode is invoked to align a base sequence of the first sequencer to a base sequence of the second sequencer with a kmer cutting and Hash alignment method, and after alignment data is obtained, error correction is performed on the base sequence of the second sequencer according to the alignment data, and the corrected base sequence is spliced with the base sequence of the first sequencer to obtain a spliced genome and corresponding variation information.

In step S3223, alternatively, the third assembling mode is invoked to splice a base sequence of the first sequencer into contig, then, error correction is performed on a base sequence of the second sequencer by means of contig, and the corrected base sequence is spliced with the base sequence of the first sequencer to obtain a spliced genome and corresponding variation information.

In step S3224, alternatively, the third assembling mode is invoked to assemble a base sequence of the first sequencer with a kmer cutting and de bruijn graph assembling method, a base sequence of the second sequencer is aligned to an assembled de bruijn graph, and scaffold is constructed according to an alignment result to obtain a spliced genome and corresponding variation information.

In the present embodiment, the third assembling mode is selected by a user after the plurality of assembling modes provided in advance are evaluated, and therefore, specific contents of the third assembling mode are dynamically changed according to the evaluation indexes selected by the user.

For example, in the present disclosure, the base sequence of the first sequencer can be assembled according to the kmer cutting and de bruijn graph assembling method, and the assembled base sequence can be spliced with the base sequence of the second sequencer. In this process, the second sequencer can correct parts of splicing errors in the first sequencer, and the first sequencer can correct sequencing errors of the second sequencer and repair a gap area for a sequencing result from the second sequencer so as to obtain the spliced genome and the corresponding variation information, which is an assembling mode. In the present disclosure, the base sequence of the first sequencer can also be aligned to the base sequence of the second sequencer according to the kmer cutting and Hash alignment method, during which m mispairing is allowed, and after the alignment data is obtained, error correction can be performed on the base sequence of the second sequencer according to the alignment data, and then, the corrected base sequence is spliced with the base sequence of the first sequencer to obtain the spliced genome and the corresponding variation information, which is also an assembling mode. In the present disclosure, the base sequence of the first sequencer can also be spliced into contig, then, error correction is performed on the base sequence of the second sequencer by means of contig, and then, the corrected base sequence is assembled to obtain the spliced genome and the corresponding variation information, which is also an assembling mode. Moreover, in the present disclosure, the base sequence of the first sequencer can also be assembled according to the kmer cutting and de bruijn graph assembling method, the base sequence of the second sequencer is aligned to the assembled de bruijn graph, and then, scaffold is constructed according to an alignment result to obtain the spliced genome and the corresponding variation information, which is also an assembling mode.

Based on this, in the present disclosure, the base sequence can be assembled at least by means of the above-mentioned four assembling modes, and the assembled base sequence can be analyzed.

In an embodiment, that an analysis mode corresponding to a species to which the to-be-analyzed sample belongs and a sequencing technology used by the target sequencer is invoked to analyze the base sequence in step S130 can further include following steps.

In step S133, if the target sequencer is the first sequencer or the second sequencer, a species tree construction mode corresponding to the species to which the to-be-analyzed sample belongs is invoked to construct a species tree corresponding to the species to which the to-be-analyzed sample belongs, and the base sequence is analyzed according to the constructed species tree.

In step S134, alternatively, if the target sequencer is the first sequencer and the second sequencer, the base sequence of the first sequencer and the base sequence of the second sequencer are transcribed to form transcriptome data, and a data alignment process and a data length, quality and integrity analysis process are performed on the transcriptome data, so as to parse a regulation mechanism for metabolic expression of the species to which the to-be-analyzed sample belongs.

In the present embodiment, when the base sequence is analyzed, the corresponding species tree can also be constructed according to the sequencing technology used by the target sequencer and the species to which the to-be-analyzed sample belongs, and the base sequence is analyzed according to the constructed species tree.

Specifically, in the present disclosure, when the phylogenetic species tree is constructed, multi-sequence alignment can be performed on raw data of the first sequencer and the second sequencer to form a homologous area (alignment of homologous loci), then, an optimal tree construction method, such as a distance conversion method, a maximum parsimony method, a maximum likelihood method and a Bayesian method is selected according to data properties to establish a substitution model, then establish an evolution tree and evaluate the evolution tree, and thus, multi-dimensional analysis is performed on the base sequence.

Further, in the present disclosure, when the target sequencer is the first sequencer and the second sequencer, the base sequence of the first sequencer and the base sequence of the second sequencer can also be transcribed to form the transcriptome data, and the data alignment process and the data length, quality and integrity analysis process can be performed on the transcriptome data so as to parse the regulation mechanism for metabolic expression of the species to which the to-be-analyzed sample belongs.

Specifically, in the present disclosure, when subsequent species evolution and genetic relationship construction are performed, and the correlation and variation characteristics among the species are analyzed, and transcriptome data alignment, and the data length, quality and integrity analysis process, species database annotation, correlation analysis, cluster analysis, abundance analysis, etc. can also be performed according to raw transcriptome data of the second generation sequencing technology and the third generation sequencing technology, and thus, the regulation mechanism for metabolic expression of the species is parsed.

In an embodiment, when the target sequencer is the first sequencer and the second sequencer, the method can further include the following step.

In step S140, if quality values of parts of loci in the base sequence or the assembled base sequence are lower than a preset quality threshold and directly affect an annotation result when a variation result of the base sequence is annotated or species annotation is performed on the assembled base sequence, the parts of loci are verified in a locus mutation manner or a short fragment mutation manner by means of a sequencer using a first generation sequencing technology.

In the present embodiment, if the quality values of the parts of loci in the base sequence are lower than the preset quality threshold and directly affect the annotation result, for example, the accuracy of the annotation result is directly lowered, when the variation result of the base sequence of the human is annotated or the species annotation is performed on the assembled base sequence of other species, locus verification can be performed on the parts of loci in the locus mutation manner or the short fragment mutation manner by means of the sequencer using the first generation sequencing technology, and thus, the quality values of the loci are improved, and then, the accuracy of the annotation result is improved.

Specifically, in the present disclosure, during annotation, the variation result is comprehensively annotated by means of the annotation and filtering of bioinformatics analysis, basic information of variation under the combined action of the second generation sequencing technology or the third generation sequencing technology, disease information, a population frequency, software prediction results and pathogenicity reports, and pathogenicity variation is selected by comprehensively considering biological hazards, genetic anastomosis, and clinical feature anastomosis. If there are still parts of loci having lower quality values and directly affecting the annotation result, verification can be performed in the locus mutation manner or the short fragment mutation manner according to a first generation sequencing result, then, Google literatures, public databases, self-developed databases, etc. are searched according to an ACMG guideline, a CNV 5-step decision rule, etc. to determine the pathogenicity classification (P/LP/VUS/LB/B) of variation, and finally, automatic report generation is achieved according to an appointed report generation rule and a template invoking rule.

A flow sequencing and analysis apparatus provided in an embodiment of the present disclosure will be described below, and the flow sequencing and analysis apparatus described hereinafter and the flow sequencing and analysis method described above can correspondingly refer to each other.

In an embodiment, as shown in FIG. 4 which is a schematic structural diagram of a flow sequencing and analysis apparatus provided in an embodiment of the present disclosure, the present disclosure further provides a flow sequencing and analysis apparatus which can include a data acquisition module 210, a sequencing module 220 and an analysis module 230 which are specifically described as follows.

The data acquisition module 210 is configured to acquire a to-be-analyzed sample, an analysis demand of the to-be-analyzed sample, and idle conditions of all current sequencers.

The sequencing module 220 is configured to determine a target sequencer according to a sample property of the to-be-analyzed sample, the analysis demand of the to-be-analyzed sample, and the idle conditions of all the current sequencers, and sequence the to-be-analyzed sample by means of the target sequencer to obtain a base sequence corresponding to the to-be-analyzed sample, where the base sequence is a binary sequence.

The analysis module 230 is configured to input the base sequence to an internal storage, and invoke an analysis mode corresponding to a species to which the to-be-analyzed sample belongs and a sequencing technology used by the target sequencer to analyze the base sequence and generate a sequencing report.

In the above-mentioned embodiment, after the to-be-analyzed sample and the corresponding analysis demand thereof and the idle conditions of all the current sequencers are acquired, the first sequencer using the second generation sequencing technology and the second sequencer using the third generation sequencing technology have different advantages, and therefore, in the present disclosure, the target sequencer can be determined in conjunction with the sample property of the to-be-analyzed sample, the analysis demand of the to-be-analyzed sample, and the idle conditions of all the current sequencers. The target sequencer can be the first sequencer or the second sequencer, or can use the first sequencer and the second sequencer for mixed sequencing. In this way, not only diversified use scenarios can be provided for users, but also the sequencing efficiency and the sequencing precision can be improved to a certain extent. Next, in the present disclosure, the to-be-analyzed sample can be sequenced by means of the target sequencer to obtain the base sequence corresponding to the to-be-analyzed sample, the base sequence is the binary sequence, that is, an ACGT base can be stored and transmitted to the internal storage by means of a binary sequence of 00, 01, 10 and 11. Then, the analysis mode corresponding to the species to which the to-be-analyzed sample belongs and the sequencing technology used by the target sequencer is directly invoked in the internal storage to analyze the base sequence and generate the sequencing report. In this way, a basecall process of sequencing and a subsequent analysis process can be linked through the internal storage, and sequencing and analysis processes of the to-be-analyzed sample are implemented by one process, so that it is simple, convenient and fast for operating staff to operate. With the linkage based on the internal storage, the analysis efficiency can also be increased, and I/O congestion can be reduced. Moreover, the base sequence in the binary coded format shortens the read and write time, more facilitates subsequent analysis process, increases the analysis efficiency of the overall process and is strong in adaptability. By means of a novel coding mode and processing in the case that existing machine parameters are basically unchanged, the service life of a machine is prolonged while the program operation efficiency is increased without affecting the sequencing and analysis accuracy

In an embodiment, the present disclosure further provides a storage medium. The storage medium has computer-readable instructions stored thereon. The computer-readable instructions, when executed by one or more processors, cause the one or more processors to perform the steps of the flow sequencing and analysis method according to any one of the above-mentioned embodiments.

In an embodiment, the present disclosure further provides a computer device. The computer device includes one or more processors and a memory. The memory has computer-readable instructions stored thereon. The computer-readable instructions, when executed by the one or more processors, implement the steps of the flow sequencing and analysis method according to any one of the above-mentioned embodiments.

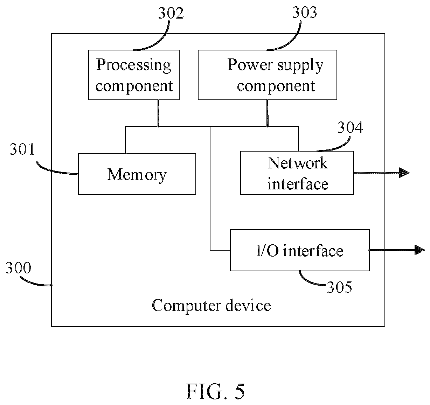

Schematically, as shown in FIG. 5 which is a schematic diagram showing an internal structure of a computer device provided in an embodiment of the present disclosure, the computer device 300 can be provided as a server. With reference to FIG. 5, the computer device 300 includes a processing component 302 and a memory resource represented by a memory 301. The processing component 302 includes one or more processors. The memory resource is configured to store an instruction, such as an application program, executable by the processing component 302. The application program stored in the memory 301 can include one or more modules with each corresponding to a set of instructions. In addition, the processing component 302 is configured to execute the instruction so that the flow sequencing and analysis method in any one of the above-mentioned embodiments is performed.

The computer device 300 can further include a power supply component 303 configured to perform power supply management on the computer device 300, a wired or wireless network interface 304 configured to connect the computer device 300 to a network, and an input/output (I/O) interface 305. The computer device 300 can operate on the basis of an operating system, such as Windows Server TM, Mac OS XTM, Unix TM, Linux TM, Free BSDTM or the like, stored in the memory 301.

It can be understood by the skilled in the art that a structure shown in FIG. 5 is only a block diagram of a partial structure relevant to the solution of the present disclosure, does not constitute a limitation on the computer device to which the solution of the present disclosure is applied, and the specific computer device can include more or fewer components than those shown in the figure, or combine some components, or have different component layouts.

Finally, it should be further noted that relational terms, such as first and second described herein, are only used to distinguish one entity or operation from another one, but do not necessarily require or imply the presence of any such actual relationship or order between these entities or operations. Moreover, terms "includes", "including" or any other variants thereof are intended to cover non-excludable inclusion, so that a process, method, article or device including a series of elements not only includes those elements, but also includes other elements not listed clearly, or further includes inherent elements of the process, method, article or device. In a case that there are no more limitations, elements defined by the word "including a..." do not exclude other same elements further existing in the process, method, article or device including the elements.

All the embodiments in the present description are described in a progressive mode, the emphasis in all the embodiments will focus on differences from other embodiments, all the embodiments can be combined as required, and the same or similar parts can refer to each other.

Due to the above-mentioned description for the disclosed embodiments, those skilled in the art can implement or use the present disclosure. A plurality of amendments to these embodiments are apparent to those skilled in the art, and general principles defined herein can be achieved in the other embodiments without departing from the spirit or scope of the present disclosure. Therefore, the present disclosure will not be limited to these embodiments shown herein, but shall accord with the widest scope consistent with the principles and novel characteristics disclosed herein.

## Claims

1. A flow sequencing and analysis method, comprising:
acquiring a to-be-analyzed sample, an analysis demand of the to-be-analyzed sample, and idle conditions of all current sequencers;
determining a target sequencer according to a sample property of the to-be-analyzed sample, the analysis demand of the to-be-analyzed sample, and the idle conditions of all the current sequencers, and sequencing the to-be-analyzed sample by means of the target sequencer to obtain a base sequence corresponding to the to-be-analyzed sample, wherein the base sequence is a binary sequence; and
inputting the base sequence to an internal storage, and invoking an analysis mode corresponding to a species to which the to-be-analyzed sample belongs and a sequencing technology used by the target sequencer to analyze the base sequence and generate a sequencing report.

2. The flow sequencing and analysis method according to claim **1,** wherein all the current sequencers comprise a first sequencer using a second generation sequencing technology and/or a second sequencer using a third generation sequencing technology.

3. The flow sequencing and analysis method according to claim 2, wherein the determining a target sequencer according to a sample property of the to-be-analyzed sample, the analysis demand of the to-be-analyzed sample, and the idle conditions of all the current sequencers comprises:
inputting the sample property of the to-be-analyzed sample and the analysis demand of the to-be-analyzed sample to a pre-configured target classification model to obtain a sequencer type, the sequencer type being outputted by the target classification model and applicable to the to-be-analyzed sample, wherein the target classification model is obtained by training with historical analysis samples comprising sample properties and analysis demands as training samples and sequencer types applicable to the training samples as sample labels; and
determining, according to the sequencer type applicable to the to-be-analyzed sample and the idle conditions of all the current sequencers, the target sequencer.

4. The flow sequencing and analysis method according to claim 3, wherein a training process for the target classification model comprises:
acquiring a plurality of historical analysis samples comprising sample properties and analysis demands and real sequencer types applicable to the plurality of the historical analysis samples, and dividing the plurality of historical analysis samples into a training set and a test set;
inputting the historical analysis samples in the training set to an initial classification model to obtain predicted sequencer types, outputted by the initial classification model, of the historical analysis samples;
training the initial classification model with a goal that the predicted sequencer types approach to the real sequencer types applicable to the historical analysis samples; and
when the initial classification model meets a preset training condition, adjusting and optimizing the trained initial classification model by using the historical analysis samples in the test set to obtain the final target classification model.

5. The flow sequencing and analysis method according to claim 2, wherein the determining a target sequencer according to a sample property of the to-be-analyzed sample, the analysis demand of the to-be-analyzed sample, and the idle conditions of all the current sequencers comprises:
comparing the sample property of the to-be-analyzed sample and the analysis demand of the to-be-analyzed sample with corresponding thresholds respectively, or comparing the analysis demand of the to-be-analyzed sample with a corresponding threshold alone; and
determining, according to a comparing result and the idle conditions of all the current sequencers, the target sequencer.

6. The flow sequencing and analysis method according to claim 5, wherein the comparing the sample property of the to-be-analyzed sample and the analysis demand of the to-be-analyzed sample with corresponding thresholds respectively or comparing the analysis demand of the to-be-analyzed sample with a corresponding threshold alone, and determining, according to a comparing result and the idle conditions of all the current sequencers, the target sequencer comprises:
if the analysis demand of the to-be-analyzed sample comprises an unlimited sequencing cost, a sequencing cost lower than a preset cost threshold, an analysis precision higher than a preset precision threshold, or a specific function detection requirement, taking both the first sequencer and the second sequencer as the target sequencer, and verifying a sequencing result from the target sequencer by means of a sequencer using a first generation sequencing technology;
if the analysis demand of the to-be-analyzed sample comprises the sequencing cost lower than the preset cost threshold, and an analysis accuracy higher than a preset accuracy threshold, determining, according to idle conditions of the first sequencers of different types, the target sequencer;
if the analysis demand of the to-be-analyzed sample comprises the sequencing cost lower than the preset cost threshold and the analysis accuracy higher than the preset accuracy threshold, and if the sample property of the to-be-analyzed sample comprises a raw data volume smaller than a preset data volume threshold, selecting a sequencer with a higher accuracy and a function meeting requirements as the target sequencer according to accuracies of the first sequencer and the second sequencer and whether functions of the first sequencer and the second sequencer meet requirements, or determining, according to idle conditions of the first sequencer and the second sequencer, the target sequencer in the case that the first sequencer and the second sequencer have the same accuracies and functions; and
if the analysis demand of the to-be-analyzed sample comprises the unlimited sequencing cost, the analysis precision lower than the preset precision threshold and a sequencing duration shorter than a preset sequencing duration, and if an analysis environment where the to-be-analyzed sample is located is a non-normal environment, selecting Nanopore in the second sequencer as the target sequencer.

7. The flow sequencing and analysis method according to claim 2, wherein the sequencing the to-be-analyzed sample by means of the target sequencer to obtain a base sequence corresponding to the to-be-analyzed sample comprises:
sequencing the to-be-analyzed sample by means of the target sequencer, and converting a sequencing result from the target sequencer into a binary sequence according to a sequencing mode of the target sequencer, to obtain the base sequence corresponding to the to-be-analyzed sample.

8. The flow sequencing and analysis method according to claim 7, wherein the sequencing the to-be-analyzed sample by means of the target sequencer and converting a sequencing result from the target sequencer into a binary sequence according to a sequencing mode of the target sequencer to obtain the base sequence corresponding to the to-be-analyzed sample comprises:
if the target sequencer is the first sequencer, directly sequencing the to-be-analyzed sample by means of the first sequencer, performing, by means of an optical device, base recognition on a fluorescence signal obtained by sequencing, and converting a base recognition result into a binary sequence in a binary coded format to obtain the base sequence corresponding to the to-be-analyzed sample; or sequencing the to-be-analyzed sample by means of the first sequencer, and performing, by means of a semiconductor chip, light intensity value detection on a fluorescence signal obtained by sequencing, and converting a light intensity signal into an electric signal in a binary coded format to obtain the base sequence corresponding to the to-be-analyzed sample;
if the target sequencer is the second sequencer, directly sequencing the to-be-analyzed sample by means of the second sequencer, and converting a current signal obtained by sequencing into a binary sequence in a binary coded format to obtain the base sequence corresponding to the to-be-analyzed sample; or sequencing the to-be-analyzed sample by means of the second sequencer, performing, by means of a semiconductor chip, light intensity value detection on a fluorescence signal obtained by sequencing, and converting a light intensity signal into an electric signal in a binary coded format to obtain the base sequence corresponding to the to-be-analyzed sample.

9. The flow sequencing and analysis method according to claim 8, wherein the optical device comprises a spectroscope and convex lenses; and
wherein the performing, by means of an optical device, base recognition on a fluorescence signal obtained by sequencing and converting a base recognition result into a binary sequence in a binary coded format comprises:
dividing, by means of the spectroscope, a beam of photons corresponding to the fluorescence signal obtained by sequencing into four beams, filtering the four beams, and converting the four filtered beams into parallel light by means of four convex lenses; and
converting the parallel light into a binary sequence by means of a CMOS camera.

10. The flow sequencing and analysis method according to claim 8, wherein the performing, by means of a semiconductor chip, light intensity value detection on a fluorescence signal obtained by sequencing and converting a light intensity signal into an electric signal in a binary coded format comprises:
performing stacked packaging on an upper layer of the semiconductor chip, and performing, by means of the packaged semiconductor chip, intelligent color filter processing on the fluorescence signal obtained by sequencing, to allow the fluorescence signal color-filtered by four colors of light filters in an intelligent color filter to fall on a specified position of each of light filters in a lower light guide pipe;
collecting the color-filtered fluorescence signal at the lower light guide pipe, and detecting a light intensity value of the color-filtered fluorescence signal by means of a photodiode in the semiconductor chip to obtain a detection result; and
converting the detection result into the electric signal in the binary coded format by means of the CMOS camera.

11. The flow sequencing and analysis method according to claim 2, wherein the invoking an analysis mode corresponding to a species to which the to-be-analyzed sample belongs and a sequencing technology used by the target sequencer to analyze the base sequence comprises:
if the species to which the to-be-analyzed sample belongs is a human having a reference genome, invoking a first analysis mode to analyze the base sequence when the target sequencer is the first sequencer, invoking a second analysis mode to analyze the base sequence when the target sequencer is the second sequencer, and invoking a third analysis mode to analyze the base sequence when the target sequencer is the first sequencer and the second sequencer; and
if the species to which the to-be-analyzed sample belongs is other species not having a reference genome, invoking a first assembling mode to assemble the base sequence when the target sequencer is the first sequencer, invoking a second assembling mode to assemble the base sequence when the target sequencer is the second sequencer, and invoking a third assembling mode to assemble the base sequence when the target sequencer is the first sequencer and the second sequencer, and analyzing the assembled base sequence.

12. The flow sequencing and analysis method according to claim 11, wherein the invoking a second analysis mode to analyze the base sequence when the target sequencer is the second sequencer comprises, when the target sequencer is the second sequencer:
performing sequence alignment, sorting, deduplication, variation detection, sequence annotation and interpretation on the base sequence to obtain the sequencing report; or
performing sequence splicing on the base sequence by using a preset sequence splicing algorithm to obtain variation information corresponding to the base sequence, and performing sequence annotation and interpretation on the variation information to obtain the sequencing report.

13. The flow sequencing and analysis method according to claim 11, wherein the invoking a third assembling mode to assemble the base sequence when the target sequencer is the first sequencer and the second sequencer and analyzing the assembled base sequence comprises, when the target sequencer is the first sequencer and the second sequencer:
determining evaluation results obtained by performing evaluation by a user on a plurality of assembling modes provided in advance according to different evaluation indexes;
selecting, according to the evaluation results, at least one assembling mode from the plurality of assembling modes provided in advance as the third assembling mode; and
invoking the third assembling mode to assemble the base sequence, and analyzing the assembled base sequence.

14. The flow sequencing and analysis method according to claim 13, wherein the invoking the third assembling mode to assemble the base sequence and analyzing the assembled base sequence comprises:
invoking the third assembling mode to assemble a base sequence of the first sequencer with a kmer cutting and de bruijn graph assembling method, and splicing the assembled base sequence with a base sequence of the second sequencer to obtain a spliced genome and corresponding variation information; or
invoking the third assembling mode to align a base sequence of the first sequencer to a base sequence of the second sequencer with a kmer cutting and Hash alignment method, and after alignment data is obtained, performing error correction on the base sequence of the second sequencer according to the alignment data, and splicing the corrected base sequence with the base sequence of the first sequencer to obtain a spliced genome and corresponding variation information; or
invoking the third assembling mode to splice a base sequence of the first sequencer into contig, performing error correction on a base sequence of the second sequencer by means of contig, and assembling the corrected base sequence to obtain a spliced genome and corresponding variation information; or
invoking the third assembling mode to assemble a base sequence of the first sequencer with a kmer cutting and de bruijn graph assembling method, aligning a base sequence of the second sequencer to an assembled de bruijn graph, and constructing scaffold according to an alignment result to obtain a spliced genome and corresponding variation information.

15. The flow sequencing and analysis method according to claim 2 or 11, wherein the invoking an analysis mode corresponding to a species to which the to-be-analyzed sample belongs and a sequencing technology used by the target sequencer to analyze the base sequence further comprises:
if the target sequencer is the first sequencer or the second sequencer, invoking a species tree construction mode corresponding to the species to which the to-be-analyzed sample belongs to construct a species tree corresponding to the species to which the to-be-analyzed sample belongs, and analyzing the base sequence according to the constructed species tree; or
if the target sequencer is the first sequencer and the second sequencer, transcribing a base sequence of the first sequencer and a base sequence of the second sequencer to form transcriptome data, and performing a data alignment process and a data length, quality and integrity analysis process on the transcriptome data, to parse a regulation mechanism for metabolic expression of the species to which the to-be-analyzed sample belongs.

16. The flow sequencing and analysis method according to claim 11, wherein the method further comprises, when the target sequencer is the first sequencer and the second sequencer:
if quality values of parts of loci in the base sequence or the assembled base sequence are lower than a preset quality threshold and directly affect an annotation result when a variation result of the base sequence is annotated or species annotation is performed on the assembled base sequence, verifying the parts of loci in a locus mutation manner or a short fragment mutation manner by means of a sequencer using a first generation sequencing technology.

17. A flow sequencing and analysis apparatus, comprising:
a data acquisition module configured to acquire a to-be-analyzed sample, an analysis demand of the to-be-analyzed sample, and idle conditions of all current sequencers;
a sequencing module configured to: determine a target sequencer according to a sample property of the to-be-analyzed sample, the analysis demand of the to-be-analyzed sample, and the idle conditions of all the current sequencers, and sequence the to-be-analyzed sample by means of the target sequencer to obtain a base sequence corresponding to the to-be-analyzed sample, wherein the base sequence is a binary sequence; and
an analysis module configured to input the base sequence to an internal storage, and invoke an analysis mode corresponding to a species to which the to-be-analyzed sample belongs and a sequencing technology used by the target sequencer to analyze the base sequence and generate a sequencing report.

18. A storage medium having computer-readable instructions stored thereon, wherein the computer-readable instructions, when executed by one or more processors, cause the one or more processors to perform steps of the flow sequencing and analysis method according to any one of claims 1 to 16.

19. A computer device comprising one or more processors and a memory, wherein the memory has computer-readable instructions stored thereon, the computer-readable instructions, when executed by the one or more processors, implementing steps of the flow sequencing and analysis method according to any one of claims 1 to 16.
